# EUROPÄISCHE PATENTANMELDUNG

(11) **EP 2 664 348 A1**
(43) Veröffentlichungstag der Anmeldung: **20.11.2013**
(21) Anmeldenummer: 12199031.1
(22) Anmeldetag: 21.12.2012
(51) Int. Cl.: A61L 9/12, A01M 1/20

(54) **Wirkstoff-Abgabevorrichtung**

(30) Priorität: 15.03.2012 DE 102012005063
(71) Anmelder: Budich International GmbH, 32120 Hiddenhausen (DE)
(72) Erfinder: Budich, Meinrad, 32609 Hüllhorst (DE)
(74) Vertreter: Beckord & Niedlich

(57) **Zusammenfassung**

Die Erfindung betrifft eine Wirkstoff-Abgabevorrichtung (1) zur Abgabe von gasförmigen Wirkstoffen, insbesondere von Duftstoffen, in die Umgebung. Diese Wirkstoff-Abgabevorrichtung (1) weist ein Gehäuse (3, 3') mit einem Gehäuseunterteil (3a, 3a') und einem Gehäuseoberteil (3b, 3b') auf, wobei das Gehäuseoberteil (3b, 3b') und das Gehäuseunterteil (3a) unter Bildung eines Spalts (17) mit einem Abstand (a, a') voneinander angeordnet sind. Weiterhin weist die Wirkstoff-Abgabevorrichtung (1) einen dem Gehäuseunterteil (3a, 3a') zugeordneten unteren Wirkstoffträger (2a, 2a') und einen dem Gehäuseoberteil (3b, 3b') zugeordneten oberen Wirkstoffträger (2b, 2b') auf, wobei der untere Wirkstoffträger (2a, 2a') und der obere Wirkstoffträger (2b, 2b') jeweils zumindest teilweise in dem Spalt (17) angeordnet sind und der obere Wirkstoffträger (2b, 2b') mit dem Gehäuseoberteil (3b, 3b') gekoppelt ist. Darüber hinaus betrifft die Erfindung ein Gehäuse (3, 3'), einen Wirkstoffträger (2a, 2b, 2a', 2b') sowie einen Wirkstoffbehälter (30) für eine derartige Wirkstoff-Abgabevorrichtung (1).

## Beschreibung

Die Erfindung betrifft eine Wirkstoff-Abgabevorrichtung zur Abgabe von gasförmigen Wirkstoffen, insbesondere Duftstoffen, in die Umgebung. Diese Wirkstoff-Abgabevorrichtung weist ein Gehäuse mit einem Gehäuseunterteil und einem Gehäuseoberteil auf, welche unter Bildung eines Spalts mit einem Abstand voneinander angeordnet sind, wobei mehrere Wirkstoffträger, welche jeweils einen Wirk- bzw. Duftstoff oder einen die Wirk- bzw. Duftstoffe abgebenden Trägerstoff aufnehmen, zumindest teilweise in dem Spalt angeordnet sind.

Eine Wirkstoff-Abgabevorrichtung der eingangs genannten Art wird zum Beispiel in der deutschen Patentanmeldung DE 10 2010 051 409 sowie der europäischen Patentanmeldung mit dem Aktenzeichen EP 11 008 739.2 beschrieben. Dabei ist wie erwähnt das Gehäuse aus einem Gehäuseunterteil und einem Gehäuseoberteil gebildet. Das Gehäuseunterteil kann eine Standfläche aufweisen, die eine kippsichere Platzierung der Wirkstoff-Abgabevorrichtung auf einer ebenen Fläche, z. B. einem Tisch, ermöglicht, während das Gehäuseoberteil als eine Art Abdeckung ausgebildet sein kann. Das Gehäuseoberteil und das Gehäuseunterteil des Gehäuses sind so zueinander angeordnet, dass sie einen Spalt mit einem Abstand, vorzugsweise einem nicht weiter reduzierbaren Mindestabstand bilden. In diesem vorzugsweise sich in einer vertikalen Ebene erstreckenden, d.h. in einer horizontalen Ebene umlaufenden, radial nach außen offenen Spalt ist zumindest teilweise ein Wirkstoffträger angeordnet. Auf einer zum Spalt weisenden Oberseite weisen die Wirkstoffträger jeweils eine Kavität auf, in welcher sich der Wirkstoff bzw. Duftstoff befindet.

Die Wirkstoffabgaberate wird unter anderem stark von der zur Verfügung stehenden Abdampfoberfläche bestimmt, an der der Wirkstoff oder der den Wirkstoff enthaltende Trägerstoff mit der Umgebungsluft in Berührung kommt. Um eine höhere Wirkstoffabgaberate zu erreichen, zum Beispiel zur Beduftung von größeren Räumen, könnte die Abdampfoberfläche vergrößert werden. Hierzu könnte das komplette Gehäuse der Wirkstoff-Abgabevorrichtung vergrößert werden, was aber aus Platzgründen und optischen Gründen nicht immer vorteilhaft ist. Eine weitere Möglichkeit besteht prinzipiell darin, mehrere Wirkstoffträger innerhalb der Wirkstoff-Abgabevorrichtung einzusetzen.

In einer in der DE 10 2010 051 409 erwähnten Variante der Wirkstoff-Abgabevorrichtung können auch mehrere Wirkstoffträger im Spalt zwischen Gehäuseunterteil und Gehäuseoberteil angeordnet sein, wobei der unterste Wirkstoffträger auf die Oberseite des Gehäuseunterteils aufgelegt wird und darüber dann die weiteren Wirkstoffträger aufgelegt werden. Die Verwendung mehrerer Wirkstoffträger dient hier in erster Linie dazu, Wirkstoffträger mit unterschiedlichen gasförmigen Wirkstoffen einzusetzen, insbesondere unterschiedlichen Duftnoten, um so verschiedene Düfte auf Wunsch miteinander kombinieren zu können. Durch die darin vorgeschlagene Konstruktion wird jedoch ein unterer Wirkstoffträger durch einen darüber liegenden Wirkstoffträger auf seiner den Duftstoff enthaltenden Oberseite abgedeckt, wodurch die Duftabgabe des unteren Wirkstoffträgers reduziert wird. Für eine stärkere Erhöhung der Wirkstoffabgaberate, gegebenenfalls auch nur eines Wirkstoffs, ist diese Konstruktion somit nicht optimal.

Es ist daher Aufgabe der vorliegenden Erfindung, eine alternative Wirkstoff-Abgabevorrichtung der eingangs genannten Art bereitzustellen, mit der auf einfache Weise eine erhebliche Erhöhung der Wirkstoffabgaberate erreicht werden kann.

Die Aufgabe wird zum einen durch eine Wirkstoff-Abgabevorrichtung nach Patentanspruch 1 und zum anderen durch ein Gehäuse nach Anspruch 13 sowie durch einen Wirkstoffträger nach Anspruch 14 und einen Wirkstoffbehälter nach Anspruch 15 gelöst.

Die erfindungsgemäße Wirkstoff-Abgabevorrichtung zur Abgabe von gasförmigen Wirkstoffen - insbesondere Duftstoffen - in die Umgebung weist ein Gehäuse und einen dem Gehäuse zugeordneten Wirkstoffträger auf, der einen die Wirk- bzw. Duftstoffe abgebenden Trägerstoff aufnehmen kann.

Die erfindungsgemäße Wirkstoff-Abgabevorrichtung weist wie eingangs erwähnt ein Gehäuse mit einem Gehäuseunterteil und einem Gehäuseoberteil auf, wobei das Gehäuseunterteil und das Gehäuseoberteil unter Bildung eines Spalts mit einem Abstand voneinander angeordnet sind. Das Gehäuse kann dabei im Wesentlichen wie das Gehäuse bei der Wirkstoff-Abgabevorrichtung gemäß der DE 10 2010 051 409 aufgebaut sein. Das heißt, das Gehäuseunterteil kann beispielsweise wieder eine Standfläche aufweisen, während das Gehäuseoberteil als eine Art Abdeckung ausgebildet ist, und der Spalt erstreckt sich (bezüglich seiner Spaltbreite) vorzugsweise in einer vertikalen Richtung, d.h. läuft in einer horizontalen Ebene um.

Zwischen dem Gehäuseunterteil und dem Gehäuseoberteil, zumindest teilweise in dem Spalt, sind die Wirkstoffträger angeordnet. Bei diesen, vorzugsweise auch scheibenförmig ausgebildeten Wirkstoffträgern handelt es sich, wie bei der DE 10 2010 051 409, jeweils um ein mechanisches Vorrichtungsteil. In oder an diesen Wirkstoffträgern ist jeweils, wie später noch genauer erläutert wird, direkt der Wirkstoff, gegebenenfalls mitsamt einem Trägerstoff bzw. Trägermaterial für den Wirkstoff untergebracht, oder die Wirkstoffbehälter nehmen - wie in der EP 11 008 739.2 genauer ausgeführt - jeweils einen Wirkstoffbehälter auf, in dem sich der Wirkstoff eventuell mit einem Trägerstoff befindet.

Im Unterschied zu dem Aufbau gemäß der DE 10 2010 051 409 ist hier jedoch dem Gehäuseunterteil ein unterer Wirkstoffträger und dem Gehäuseoberteil ein oberer Wirkstoffträger zugeordnet und der obere Wirkstoffträger ist mit dem Gehäuseoberteil gekoppelt.

Durch die Zuordnung der beiden Wirkstoffträger jeweils zu dem Gehäuseoberteil beziehungsweise Gehäuseunterteil und die Kopplung des oberen Wirkstoffträgers mit dem Gehäuseoberteil, ist es nicht mehr erforderlich, dass die Wirkstoffträger im Betrieb direkt aufeinander liegen und somit der obere Wirkstoffträger den unteren Wirkstoffträger abdeckt und die Abdampfung des darin befindlichen Wirkstoffs behindert. Dennoch ist die erfindungsgemäße Wirkstoff-Abgabevorrichtung nach wie vor - wie die Wirkstoff-Abgabevorrichtung nach der DE 10 2010 051 409 oder der EP 11 008 739.2 - einfach aufgebaut und besteht nur aus wenigen Teilen. Der Wirkstoffträger ist bei der Montage der Wirkstoff-Abgabevorrichtung auf einfache Weise montierbar und kann je nach Ausführungsbeispiel so ausgestaltet sein, dass er später werkzeuglos leicht entnehmbar bzw. austauschbar oder neu befüllbar ist, wenn der Wirkstoffvorrat erschöpft ist.

Durch den erfindungsgemäßen Aufbau kann also die Wirkstoffabgaberate eines Wirkstoffs erheblich erhöht werden, wenn in beiden Wirkstoffträgern derselbe Wirkstoff verwendet wird. Es ist aber im Rahmen der Erfindung auch möglich, dass unterschiedliche gasförmige Wirkstoffe, insbesondere unterschiedliche Duftnoten, eingesetzt werden.

Die abhängigen Ansprüche und die weitere Beschreibung enthalten besonders vorteilhafte Ausgestaltungen und Weiterbildungen der Erfindung, wobei insbesondere die Ansprüche einer Kategorie auch analog den Ansprüchen einer der anderen Kategorien weitergebildet sein können.

Besonders bevorzugt grenzt der untere Wirkstoffträger an eine zum Gehäuseoberteil weisende, d. h. an den Spalt angrenzende Oberseite des Gehäuseunterteils an oder greift in diese ein und der obere Wirkstoffträger grenzt an eine zum Gehäuseunterteil weisende, d.h. wiederum an den Spalt angrenzenden Unterseite des Gehäuseoberteil an oder greift in diese ein.

Ein besonders simpler Aufbau ergibt sich dabei, wenn der betreffende Wirkstoffträger im Wesentlichen ausschließlich in dem Spalt zwischen dem Gehäuseoberteil und dem Gehäuseunterteil angeordnet ist und sich nur geringfügig in das Gehäuseunterteil bzw. das Gehäuseoberteil erstreckt. Dies erlaubt, je nach Ausführungsvariante, einen besonders einfachen Wechsel des Wirkstoffträgers.

Zur Kopplung mit dem Gehäuseoberteil ist der obere Wirkstoffträger vorzugsweise an der Unterseite des Gehäuseoberteils mit diesem verbunden. Bei einer bevorzugten Variante handelt es sich um eine lösbare Verbindung.

Dabei kann der obere Wirkstoffträger besonders bevorzugt formschlüssig mit dem Gehäuseoberteil verbunden sein. Beispielsweise kann der obere Wirkstoffträger an einer zur Unterseite des Gehäuseoberteils weisenden Anschlussseite mechanische Verbindungselemente, vorzugsweise Rastelemente, aufweisen, welche mit mechanischen Verbindungselementen, vorzugsweise Rastelementen, an oder im Gehäuseoberteil zusammenwirken. Beispiele für solche Verbindungselemente oder Rastelemente sind eine oder mehrere in geeigneter Weise angeordnete Rastnasen, an einem äußeren Rand eines Anschlussstücks des Wirkstoffträgers teilweise oder vollständig umlaufende Wulste, Sicken etc.

Eine einfache Lösbarkeit der Kopplung, beispielsweise für einen Austausch des Wirkstoffträgers, lässt sich dadurch realisieren, dass die Verbindungselemente jeweils so ausgebildet und angeordnet sind bzw. zusammenwirken, dass der obere Wirkstoffträger in einer ersten Drehstellung relativ zum Gehäuseoberteil (bezüglich einer Drehung um eine zwischen dem Gehäuseoberteil und dem Gehäuseunterteil verlaufende Drehachse) am Gehäuseoberteil gehalten wird und in einer zweiten Drehstellung von diesem lösbar ist. Typische Beispiele hierfür sind Gewindeabschnitte, Wulste, Rillen bzw. Sicken nur auf Teilabschnitten entlang des Umfangs etc. (wie dies später anhand eines Ausführungsbeispiels noch genauer erläutert wird) oder eine Art Bajonettverschlussverbindung.

Zur Aufnahme des Wirkstoffs weisen bevorzugt der untere Wirkstoffträger und der obere Wirkstoffträger jeweils, vorzugsweise in ihren im montierten Zustand zueinander weisenden Seiten, eine Kavität zur Aufnahme des Wirkstoffs und gegebenenfalls eines den Wirkstoff enthaltenden Trägerstoffs und/oder zur Aufnahme eines Wirkstoffbehälters auf, welcher den Wirkstoff und gegebenenfalls den Wirkstoff enthaltenden Trägerstoff enthält. Genauere Ausführungsbeispiele hierzu werden später noch erläutert. Dabei kann der Wirkstoffträger mitsamt der Kavität beispielsweise durch Tiefziehen von Kunststoff oder durch Spritzguss gefertigt werden.

Vorzugsweise sind das Gehäuseoberteil und das Gehäuseunterteil sowie der Wirkstoffträger in zumindest einer ersten Position (Ausgangsposition) so angeordnet, dass der untere Wirkstoffträger und der obere Wirkstoffträger in Kontakt stehen, d. h. an den zueinander weisenden Seiten (der Unterseite des oberen Wirkstoffträgers und der Oberseite des der unteren Wirkstoffträger) aneinander liegen. Somit weist die Wirkstoff-Abgabevorrichtung in dieser ersten Position einen eng geschichteten sandwichartigen Aufbau mit zwei zwischen dem Gehäuseoberteil und dem Gehäuseunterteil angeordneten Wirkstoffträgern auf, wobei z. B. die Kavitäten der Wirkstoffträger eng aufeinander liegen können und dadurch die Abgabe von Wirkstoff vollständig unterbunden oder reduziert ist.

Dabei kann die Wirkstoff-Abgabevorrichtung eine in dieser ersten Position unverschlossene Ausdampföffnung in den Gehäuseteilen und/oder den Wirkstoffträgern zum Austritt der gasförmigen Wirkstoffe aufweisen, um eine Mindestabdampfrate zu gewährleisten. Diese Ausdampföffnung kann beispielsweise durch eine Anzahl von (d. h. ein oder mehrere) Luftströmungslöchern bzw. Aussparungen im Gehäuseoberteil und/oder im Gehäuseunterteil und/oder im Wirkstoffträger gebildet sein. Für genauere Ausführungsbeispiele hierzu wird auf die EP 11 008 739.2 verwiesen. Abhängig von der genauen Ausführungsform mit den zusätzlichen Luftströmungslöchern bzw. Aussparungen in den Gehäuseteilen bzw. im Wirkstoffträger kann es dann sinnvoll sein, die obere Scheibe mit der den Wirkstoff enthaltenden Kavität nach oben und/oder die untere Scheibe mit der den Wirkstoff enthaltenden Kavität nach unten auszurichten und/oder zumindest eine der beiden Scheiben beidseitig (d.h. auf der oberen und unteren Flachseite) mit einer Kavität mit Wirkstoff zu versehen.

Um die Abdampfrate bei Bedarf erhöhen zu können, kann der Abstand vom Gehäuseoberteil zu dem Gehäuseunterteil durch Verlagern des Gehäuseoberteils relativ zum Gehäuseunterteil entlang einer Verbindungsachse variiert werden, die zwischen Gehäuseunterteil und Gehäuseoberteil verläuft und das Gehäuseoberteil mit dem Gehäuseunterteil verbindet. Hierdurch wird die Breite des radial nach außen offenen Spaltes zwischen Gehäuseunterteil und Gehäuseoberteil entlang der Verbindungsachse erhöht und damit die Gesamtfläche der Ausdampföffnung vergrößert. Es sind also das Gehäuseoberteil und das Gehäuseunterteil derart miteinander verbunden, dass ausgehend von einer ersten Position bzw. Ausgangsposition ein Spalt mit einer Mindestbreite vergrößert werden kann. Dabei wird, da der obere Wirkstoffträger mit dem Gehäuseoberteil gekoppelt ist, automatisch der Spalt zwischen dem unteren Wirkstoffträger und dem oberen Wirkstoffträger vergrößert. So kann z. B. bei einer Verwendung von Wirkstoffträgern, welche auf den zu einander weisenden Seiten jeweils eine den Wirkstoff enthaltende Kavität enthalten, die Wirkstoff abgebende Oberfläche (die Abdampfoberfläche) im Verhältnis zu der Wirkstoff-Abgabevorrichtung gemäß der DE 10 2010 051 409 mit nur einem Wirkstoffträger bei nahezu gleichen Außenabmessungen der gesamten Wirkstoff-Abgabevorrichtung verdoppelt werden.

Hierzu können das Gehäuseunterteil und das Gehäuseoberteil vorzugsweise derart miteinander durch eine Gehäuseführung gekoppelt sein, dass eine lineare Bewegung des Gehäuseoberteils relativ zum Gehäuseunterteil entlang der Verbindungsachse durchführbar ist. Dies bewirkt, dass der Spalt bei einem Verlagern des Gehäuseoberteils in Bezug zum Gehäuseunterteil an all seinen Positionen gleichmäßig vergrößert wird, d. h. die zueinander weisenden Flächen der Wirkstoffträger bleiben trotz der Verlagerung parallel zueinander. Insbesondere können auch hier bevorzugt das Gehäuseunterteil und das Gehäuseoberteil so gekoppelt sein, dass eine Drehbewegung des Gehäuseoberteils und/oder Gehäuseunterteils um die Verbindungsachse zwangsläufig in eine lineare Bewegung des Gehäuseoberteils relativ zum Gehäuseunterteil entlang der Verbindungsachse umgewandelt wird, wobei die Drehbewegung und die gewünschte lineare Bewegung zu einer Art Schraubenbewegung überlagern. Dabei erlaubt eine derartige Kopplung des Gehäuseunterteils mit dem Gehäuseoberteil eine besonders genaue Einstellung der Breite des gewünschten Spaltes. Für die konkreten Ausgestaltungsmöglichkeiten einer solchen Kopplung wird auf die DE 10 2010 051 409 und die EP 11 008 739.2 verwiesen. Ebenso sind aber auch die anderen in diesen Voranmeldungen genannten Ausführungsformen möglich.

Bei einer bevorzugten Variante ist ein Einsetzen eines Wirkstoffträgers in das Gehäuse bzw. das Auswechseln eines Wirkstoffträgers oder eines Wirkstoffbehälters im Wirkstoffträger nach Erschöpfung des gasförmigen Wirkstoffs durch ein Verlagern des Wirkstoffträgers relativ zum Gehäuse quer zur Verbindungsachse möglich. Im einfachsten Fall wird also der Wirkstoffträger entlang einer Montageachse bewegt, die senkrecht zur Verbindungsachse des Gehäuseunterteils und des Gehäuseoberteils verläuft. Dies erlaubt ein besonders einfaches Einsetzen bzw. einen Austausch des Wirkstoffträgers bzw. Wirkstoffbehälters. Alternativ ist es auch möglich, das Gehäuseoberteil vollständig vom Gehäuseunterteil zu trennen und den Wirkstoffträger durch eine Bewegung entlang der Verbindungsachse des Gehäuseunterteils und des Gehäuseoberteils in das Gehäuse herauszunehmen oder einzufügen und anschließend das Gehäuseunterteil mit dem Gehäuseoberteil wieder zu verbinden.

Um das Austauschen eines neuen Wirkstoffträgers oder eines darin angeordneten Wirkstoffbehälters nach Verbrauch des Wirkstoffs zu erleichtern, weist die Wirkstoff-Abgabevorrichtung vorzugsweise eine Wirkstoffträgerführung auf, um den Wirkstoffträger beim Einschieben in den Spalt zu führen. Ferner weist die Wirkstoff-Abgabevorrichtung einen Anschlag auf, um diese Einführbewegung zu stoppen. Dabei ergibt sich ein besonders einfacher Aufbau, wenn diese Wirkstoffträgerführung und dieser Anschlag mit der Gehäuseführung des Gehäuses zusammenwirken, die eine Verlagerung des Gehäuseunterteils und des Gehäuseoberteils relativ zueinander erlauben, wie dies in der DE 10 2010 051 409 beschrieben ist. Das heißt, dass die Gehäuseführung eine Doppelfunktion übernimmt, nämlich zum einen eine Führung des Gehäuseoberteils in Bezug zum Gehäuseunterteil entlang der Verbindungsachse und zum anderen, zusammen mit der Wirkstoffträgerführung, eine Führung des Wirkstoffträgers entlang einer Montageachse bei einem Einschieben des Wirkstoffträgers in den Spalt, z. B. senkrecht zur Verbindungsachse. Eine besonders ästhetische Gestaltung des Gehäuses wird erreicht, wenn die Gehäuseführung, z. B. die Verbindungsachse, im Inneren des Gehäuseunterteils und/oder des Gehäuseoberteils angeordnet ist.

Eine besonders materialsparende und leichte Ausbildung des Gehäuseunterteils und/oder des Gehäuseoberteils kann erreicht werden, wenn das Gehäuseunterteil und/oder das Gehäuseoberteil als offene Hohlkörper ausgebildet sind. Zum Beispiel können das Gehäuseunterteil und/oder das Gehäuseoberteil kalottenförmig ausgebildet sein bzw. die Gestalt von hohlen Halbkugeln aufweisen. Somit weisen das Gehäuseunterteil und das Gehäuseoberteil einen Innenraum auf, in dem dann die Gehäuseführung bzw. die Verbindungsachse angeordnet sein kann. Dies erlaubt ferner eine besonders einfache Fertigung des Gehäuseunterteils und des Gehäuseoberteils aus Kunststoff mittels Spritzguss.

Wie in der DE 10 2010 051 409 erläutert, ist eine besonders einfache Montage durch Zusammenführen des Gehäuseunterteils mit dem Gehäuseoberteil sowie einem oder mehreren Wirkstoffträgern möglich, wenn die Gehäuseführung zweigeteilt ausgebildet ist, d. h. einen ersten Abschnitt und einen zweiten Abschnitt aufweist. Dabei kann der erste Abschnitt z. B. dem Gehäuseunterteil und der zweite Abschnitt z. B. dem Gehäuseoberteil zugeordnet sein. Die Länge des ersten und/oder zweiten Abschnitts kann derart gewählt sein, dass sie sich in Richtung der Verbindungsachse aus dem Gehäuseunterteil und/oder Gehäuseoberteil erstrecken. Wenn z. B. das Gehäuseunterteil und/oder das Gehäuseoberteil als hohle Halbkugeln ausgebildet sind, ist in diesem Fall die Länge des ersten und/oder zweiten Abschnitts größer als der Radius der Halbkugel.

Der Wirkstoff selbst kann z. B. in der oben genannten Kavität in einem Trägermaterial bzw. Trägerstoff wie einem Vlies, sonstigem geeigneten Fasermaterial oder einem anderen porösen Material aufgenommen sein. Die Kavität kann z. B. mittels einer gaspermeablen (semipermeablen) Membran verschlossen sein, die nur einen gasförmigen Wirkstoff hindurch treten lässt, ansonsten aber z. B. einen ungewünschten Eintritt von Flüssigkeiten in die Kavität oder einen Austritt einer Wirkstoffträgerflüssigkeit aus der Kavität verhindert. Insbesondere in diesem Fall kann also auch ein flüssiger Trägerstoff bzw. verflüssigter Wirkstoff eingesetzt werden. Ebenso ist es möglich, die Kavität mit einem bereits gasförmigen Wirkstoff zu füllen, wobei die Kavität dann durch eine Membran verschlossen ist, die nur eine bestimmte Durchlassrate für den Wirkstoff erlaubt.

Bei einer bevorzugten Variante ist in der Kavität ein gelartiger Trägerstoff aufgenommen, der den gasförmigen Wirkstoff, z. B. Duftstoffe, freisetzt. Dies erlaubt eine besonders einfache Handhabung des Wirkstoffträgers, da durch den gelartigen Trägerstoff ein unbeabsichtigter Austritt des Wirkstoffs, insbesondere beim Einsetzen des Wirkstoffträgers in den Spalt des Gehäuses, ausgeschlossen ist. In diesem Fall könnte insbesondere darauf verzichtet werden, dass die Kavität mit einer semipermeablen Membran verschlossen ist, wobei die Verwendung einer solchen Membran aber auch bei einem Gel nicht ausgeschlossen ist.

Bei einer weiteren bevorzugten Variante wird am Wirkstoffträger, besonders bevorzugt in der Kavität, ein mit dem Wirkstoff und gegebenenfalls dem Trägermaterial gefüllter Wirkstoffbehälter angeordnet. Dies hat den Vorteil, dass bei verbrauchtem Wirkstoff nur der Wirkstoffbehälter ausgewechselt werden muss, was Ressourcen schonender und kostengünstiger ist. Dabei kann vorzugsweise auch der Wirkstoffbehälter mit einer gasdurchlässigen Membran verschlossen sein und zum Beispiel mit einem flüssigen oder gasförmigen Wirkstoff bzw. Trägerstoff gefüllt sein. Ebenso kann aber der Wirkstoffbehälter auch offen sein und z. B. mit einem wirkstoffhaltigen Gel oder dergleichen gefüllt sein.

Der Wirkstoffbehälter kann relativ dünnwandig sein, vorzugsweise aus Tiefziehfolie hergestellt werden. Der Wirkstoffträger kann auch mehrere Kammern mit verschiedenen Wirkstoffen, z. B. unterschiedlichen Duftnoten, umfassen.

Die Außenmaße des Wirkstoffbehälters sind vorzugsweise an die Kavität angepasst. Besonders bevorzugt befinden sich am Wirkstoffträger, insbesondere in der Kavität des Wirkstoffträgers, und/oder am Wirkstoffbehälter Halteelemente, durch die der Wirkstoffbehälter gut am Wirkstoffträger, z. B. in der Kavität, festgehalten wird. Besonders bevorzugt weisen sowohl der Wirkstoffträger als auch der Wirkstoffbehälter zusammenwirkende Halteelemente auf. Beispielsweise kann in der Kavität des Wirkstoffträgers ein nach innen ragender Vorsprung, insbesondere ein Wulst oder dergleichen, angeordnet sein, um eine Klemmung des Wirkstoffbehälters in der Kavität zu erreichen. Am Wirkstoffbehälter selber kann ein hierzu passender Vorsprung oder eine passende Ausnehmung, beispielsweise eine Sicke, angeordnet sein, welche mit dem Vorsprung in der Kavität zusammen wirkt. Umgekehrt kann auch die Kavität eine Ausnehmung aufweisen, die mit einem Vorsprung am Wirkstoffbehälter zusammen wirkt.

Um eine besonders einfache Handhabung und Lagerung von Wirkstoffträgern zu ermöglichen, ohne dass es zu einem vorzeitigen Austritt der Wirkstoffe kommt, sind die Kavität oder der Wirkstoffbehälter vor Inbetriebnahme gasundurchlässig verschlossen, z. B. mittels eines gasundurchlässigen entfernbaren Schutzdeckels, der gegebenenfalls wiederum die gasdurchlässige Membran abdeckt. Dabei kann der gasundurchlässige Schutzdeckel als Folie ausgebildet sein, die z. B. mittels einer lösbaren Klebeverbindung am Wirkstoffträger derart angebracht ist, dass die Kavität bzw. der Wirkstoffbehälter gasundurchlässig verschlossen ist. Anstelle der Klebeverbindung kann auch eine Press- oder Rastverbindung vorgesehen sein, mit der z. B. ein im Vergleich zu einer Folie steiferer Verschlussdeckel die Kavität verschließt.

Das Gehäuseoberteil und das Gehäuseunterteil sowie der Wirkstoffträger und/oder der Wirkstoffbehälter können im Prinzip eine beliebige äußere Form aufweisen. Vorzugsweise sind die Wirkstoffträger bezüglich ihrer äußeren Kontur an die äußere Kontur des Gehäuseoberteils und des Gehäuseunterteils im Bereich des Spaltes angepasst. Eine besonders optisch ansprechende Gestaltung des Gehäuses ergibt sich, wenn das Gehäuseunterteil und das Gehäuseoberteil jeweils angrenzend an den Spalt senkrecht zur Längsrichtung der Verbindungsachse einen kreisförmigen Außen-Querschnitt aufweisen. Dabei weisen die kreisförmigen Außen-Querschnitte jeweils den gleichen Durchmesser auf. Durch diese Gestaltung des Gehäuseunterteils, des Gehäuseoberteils und des Wirkstoffträgers wird insbesondere eine im Wesentlichen rotationssymmetrische Gestaltung, z. B. Kugelform, ohne unästhetische Formabweichung, wie z. B. Stufen, möglich, auch wenn der Spalt durch eine Drehbewegung des Gehäuseoberteils in Bezug zum Gehäuseunterteil vergrößert wird.

Die optimale Wahl der Maße des Gehäuses und der Wirkstoffträger hängt sowohl vom Einsatzzweck und -ort, von der Art des Wirkstoffs, von der gewählten Wirkstoffrate und Wirkstoffmenge und auch von der gewünschten Ästhetik ab. Vorzugsweise sind das Gehäuse und die Wirkstoffträger so dimensioniert, dass der Durchmesser des Außen-Querschnitts der Gehäuseteile angrenzend an den Spalt senkrecht zur Längsrichtung der Verbindungsachse sowie der Außen-Durchmesser des Wirkstoffträgers jeweils zwischen 5 cm und 20 cm, besonders bevorzugt zwischen 7 cm und 12 cm beträgt. Die Dicke (bzw. Höhe) der scheibenförmigen Wirkstoffträger kann vorzugsweise 2 mm bis 15 mm betragen, wobei die Füllhöhe in der Kavität vorzugsweise zwischen 1 mm und 14 mm beträgt. Die Kopplung und Ausbildung der Gehäuseteile ist vorzugsweise so, dass die mögliche Maximalbreite des Spalts, d. h. bei ganz hochgefahrenem oberem Gehäuseteil, zwischen 5 mm und 40 mm liegt. Vorzugsweise beträgt in dieser Stellung die Öffnung des umlaufenden Spalts zwischen den eingesetzten Wirkstoffträgern bis zu 20 mm und mehr.

Die Erfindung wird im Folgenden unter Hinweis auf die beigefügten Figuren anhand von Ausführungsbeispielen noch einmal näher erläutert. Hieraus ergeben sich auch weitere Einzelheiten und Vorteile der Erfindung. Es zeigen:
- Figur 1: eine schematische Seitenansicht einer erfindungsgemäßen Wirkstoff-Abgabevorrichtung gemäß einem ersten Ausführungsbeispiel in einer geschlossenen Stellung,
- Figur 2: die Wirkstoff-Abgabevorrichtung wie in Figur 1, jedoch nun in einer geöffneten Stellung,
- Figur 3: eine perspektivische Darstellung eines unteren Wirkstoffträgers gemäß einem ersten Ausführungsbeispiel,
- Figur 4: eine perspektivische Explosionsdarstellung (wobei jedoch nur der untere Wirkstoffträger dargestellt ist) einer erfindungsgemäßen Wirkstoff-Abgabevorrichtung gemäß den Figuren 1 und 2,
- Figur 5: eine Draufsicht und Seitenansicht des Gehäuseunterteils des Gehäuses der Wirkstoff-Abgabevorrichtung gemäß dem ersten Ausführungsbeispiel,
- Figur 6: eine Draufsicht und Seitenansicht eines Gehäuseoberteils des Gehäuses der Wirkstoff-Abgabevorrichtung gemäß dem ersten Ausführungsbeispiel,
- Figur 7: eine Explosionsdarstellung einer erfindungsgemäßen Wirkstoff-Abgabevorrichtung gemäß den Figuren 1 und 2 von der Seite bzw. im Teilschnitt,
- Figur 8: eine Draufsicht auf einen Wirkstoffträgers gemäß Figur 7,
- Figur 9: eine Explosionsdarstellung einer erfindungsgemäßen Wirkstoff-Abgabevorrichtung gemäß einem zweiten Ausführungsbeispiel von der Seite bzw. im Teilschnitt,
- Figur 10: eine Draufsicht auf einen Wirkstoffträger gemäß Figur 9 und
- Figur 11: eine Draufsicht und eine Seitenansicht eines Wirkstoffbehälters für einen Wirkstoffträger gemäß Figur 12.

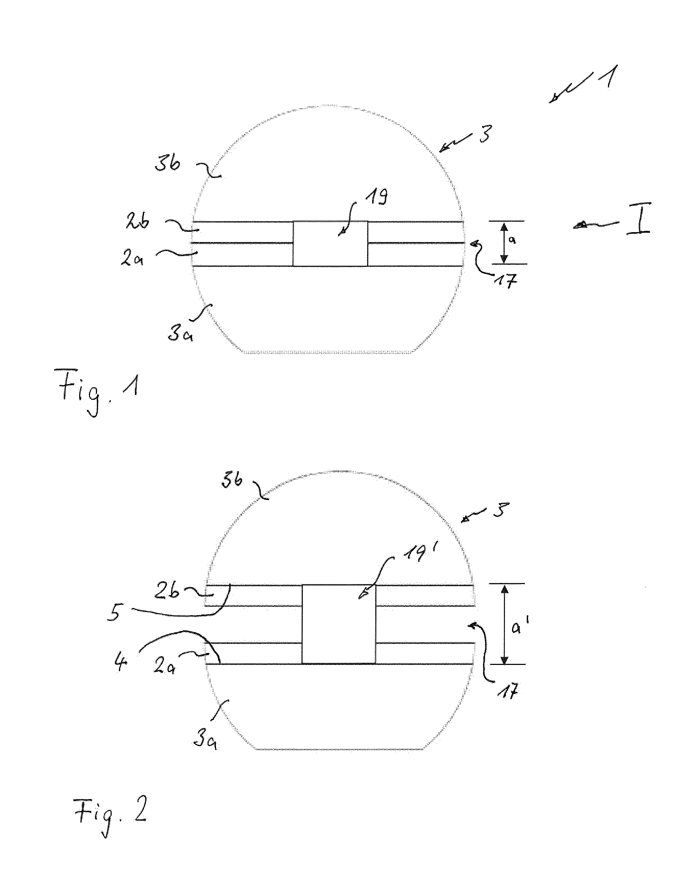

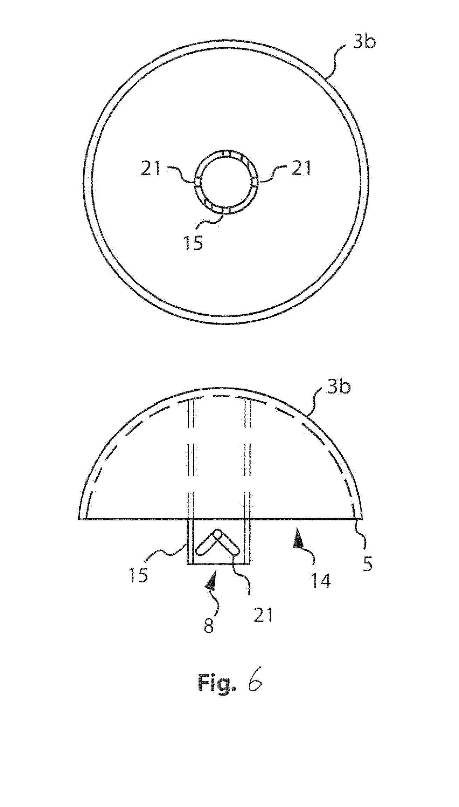

Es wird zunächst auf die Figuren 1 bis 6 Bezug genommen.

Die darin gezeigte Wirkstoff-Abgabevorrichtung 1 besteht aus einem Gehäuse 3, das aus einem Gehäuseunterteil 3a und einem Gehäuseoberteil 3b gebildet ist. Das Gehäuseunterteil 3a weist eine Standfläche 18 auf, mit der die Wirkstoff-Abgabevorrichtung 1 auf einer flachen Fläche, wie z. B. einer Tischplatte, abgestellt werden kann. Das Gehäuseunterteil 3a und das Gehäuseoberteil 3b sind außenseitig halbkugelförmig ausgebildet, so dass zusammengefügt das Gehäuse 3 eine nahezu kugelförmige Gestalt bis auf die Abflachung zur Bildung der Standfläche 18 aufweist.

Das Gehäuseoberteil 3b und das Gehäuseunterteil 3a sind in Figur 1 in einer Ausgangsposition I mit einem Abstand a voneinander angeordnet, so dass zwischen dem Gehäuseunterteil 3a und dem Gehäuseoberteil 3b ein Spalt 17 gebildet ist. In diesem Spalt 17 sind ein unterer Wirkstoffträger 2a und ein oberer Wirkstoffträger 2b eingesetzt.

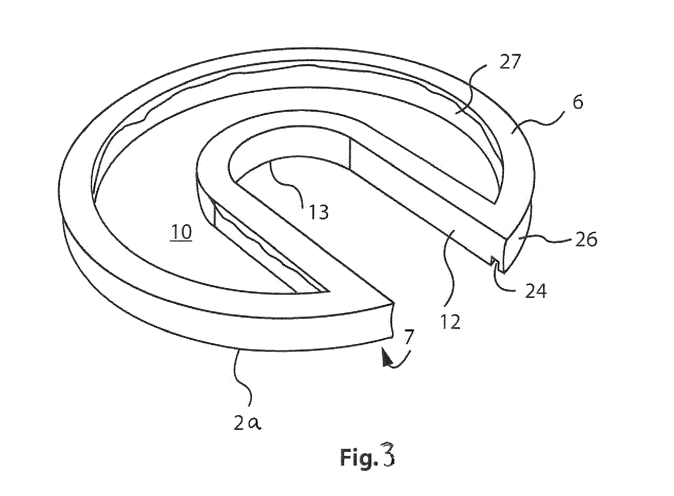

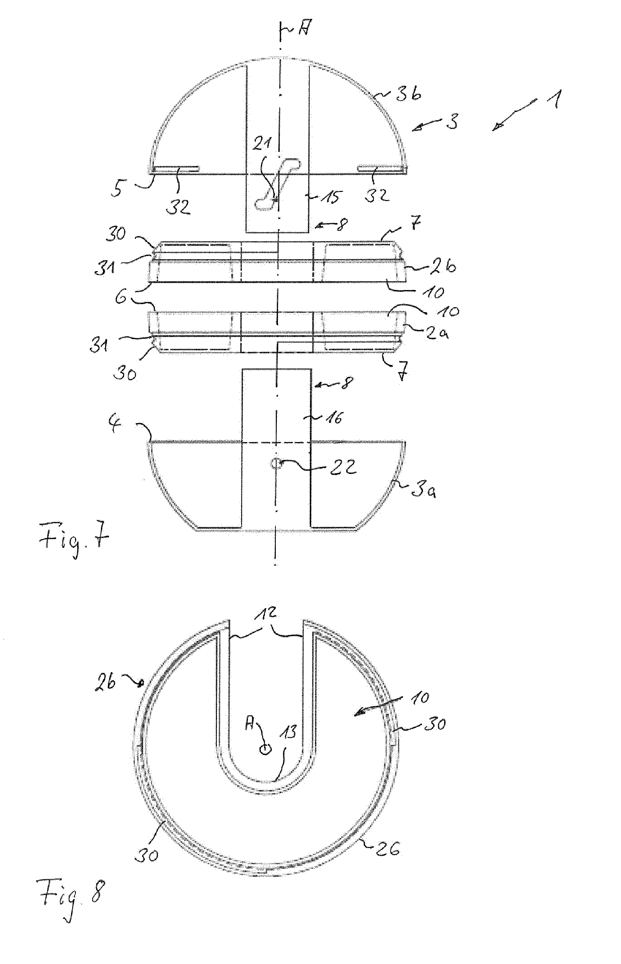

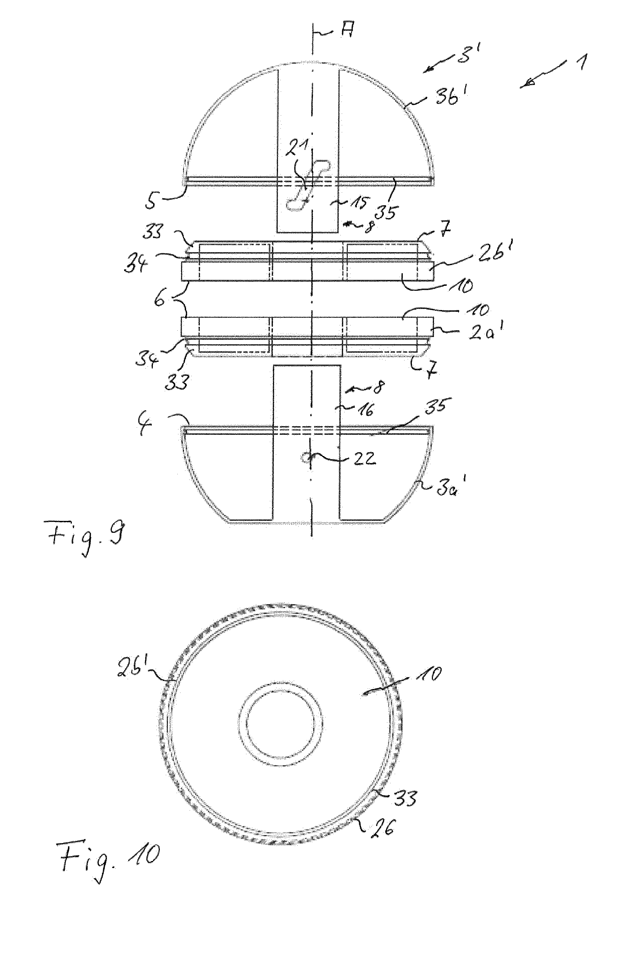

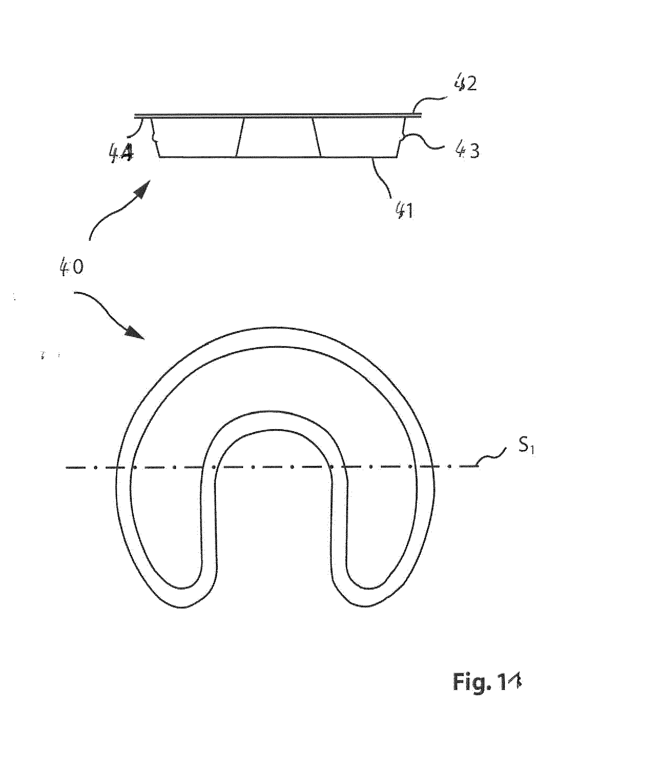

Die Wirkstoffträger 2a, 2b weisen im vorliegenden Ausführungsbeispiel jeweils an einer Wirkstoffabgabeseite 6, welche der am Gehäuseunterteil 3a bzw. Gehäuseoberteil 3b anliegenden Anschlussseite 7 des Wirkstoffträgers 2a, 2b gegenüberliegt, eine Kavität 10 auf, die mit einem gelförmigen Trägerstoff 27 gefüllt ist, der z. B. Duftstoffe an die Raumluft abgeben kann. Dies ist für den unteren Wirkstoffträger 3b in Figur 3 gut erkennbar. Der obere Wirkstoffträger 3b ist vom Grundprinzip her genauso aufgebaut, nur dass in einem in die Wirkstoff-Abgabevorrichtung 1 montierten Zustand die Kavität 10 des oberen Wirkstoffträgers 2b nach unten weist und Verbindungselemente zur Verbindung des Wirkstoffträgers 2b mit dem Gehäuseoberteil 3b am Wirkstoffträger 2b angeordnet sind (vgl. die Figuren 7 und 9). Alternativ kann auch in der Kavität 10 ein Textil oder ein Vlies zur Aufnahme eines z. B. flüssigen Wirkstoffs angeordnet sein, oder es kann sich um einen Wirkstoffträger 2' mit einem darin angeordneten Wirkstoffbehälter 30 handeln, wie er später noch anhand der Figur 11 näher erläutert wird, sofern diese auf dem oberen Wirkstoffträger 2b gehalten werden.

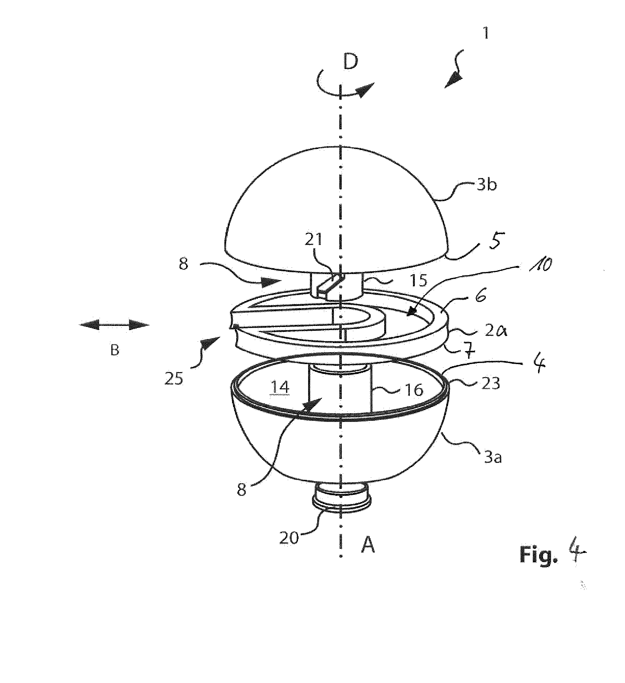

In der in Figur 1 gezeigten Ausgangsposition I liegen die Kavitäten 10 des oberen und des unteren Wirkstoffträgers 2a, 2b direkt aufeinander, so dass die Wirkstoff-Abgabevorrichtung 1 verschlossen ist und daher im Wesentlichen keinen Wirkstoff bzw. Duftstoff freigibt. Die gasförmigen Wirkstoffe können jedoch in der Ausgangsposition I aus einer durch eine Aussparung im Wirkstoffträger 2a, 2b gebildete Ausdampföffnung 19 entweichen, sofern der untere Wirkstoffträger 2a und der obere Wirkstoffträger 2b relativ zueinander um eine Verbindungsachse A (dargestellt in Figur 4) etwas verdreht werden, so dass die Aussparungen der Wirkstoffträger 2a, 2b nicht mehr deckungsgleich übereinander liegen. In diesem Fall ist auch in der Ausgangsposition I eine geringe Wirkstoffabgabe möglich, sofern dies gewünscht ist.

Um die Abdampfrate zu erhöhen, kann die Ausdampföffnung 19 vergrößert werden. Hierzu wird das Gehäuseoberteil 3b in eine - in den Figuren 2 und 3 dargestellte - zweite Position II mit einem vergrößerten Abstand a' gebracht, in der der Spalt 17 vergrößert ist und nunmehr die Ausdampföffnung 19' um einen ringförmigen Abschnitt erweitert ist. Da der obere Wirkstoffträger 2b mit dem Gehäuseoberteil 3b gekoppelt ist, wird dieser mit nach oben bewegt, so dass auch zwischen den beiden Wirkstoffträgern 2a, 2b der Spalt größer wird. Um das Gehäuseoberteil 3b aus der Ausgangsposition I in die Position II zu bringen, wird das Gehäuseoberteil 3b entlang einer Verbindungsachse A (Figur 4) verlagert.

Zugleich ist in dieser Position II ein Wechsel des Wirkstoffträgers 2 möglich, wenn sich der Vorrat an gasförmigen Wirkstoffen erschöpft hat. Hierzu wird der Wirkstoffträger 2 u. a. entlang einer (virtuellen) Montageachse B aus dem Spalt bewegt, die senkrecht zu der Verbindungsachse A verläuft.

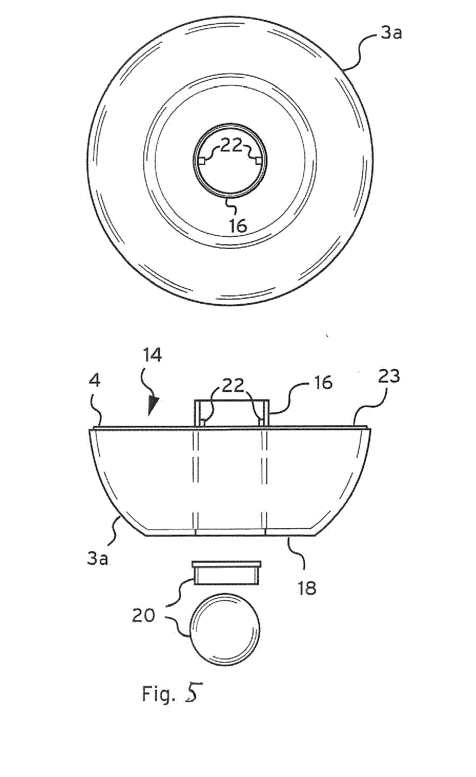

Sowohl das Gehäuseunterteil 3a als auch das Gehäuseoberteil 3b sind als Hohlkörper bzw. kalottenförmig ausgebildet, d. h. das Innere des Gehäuses 3 ist hohl (siehe Figuren 4 bis 6). Um das Verlagern des Gehäuseoberteils 3b in Bezug zum Gehäuseunterteil 3a zu ermöglichen, sind das Gehäuseoberteil 3b und das Gehäuseunterteil 3a durch eine Gehäuseführung 8 miteinander verbunden. Die Gehäuseführung 8 ist in dem Gehäuseinneren 14 des Gehäuses 3, d. h. des Gehäuseunterteils 3a und des Gehäuseoberteils 3b, angeordnet. Sie ist dabei zweigeteilt ausgebildet, d. h. die Gehäuseführung 8 weist einen ersten Abschnitt 15, der dem Gehäuseoberteil 3b zugeordnet ist, und einen zweiten Abschnitt 16 auf, der dem Gehäuseunterteil 3a zugeordnet ist. Die beiden Abschnitte 15, 16 sind als zylinderartige, rohrförmige Abschnitte ausgebildet, wobei der dem Gehäuseunterteil 3a zugeordnete Abschnitt 16 einen größeren Durchmesser aufweist als der dem Gehäuseoberteil 3b zugeordnete Abschnitt 15, so dass sie zusammen eine teleskopartige Führung zur Verlagerung des Gehäuseoberteils 3b in Bezug zum Gehäuseunterteil 3a bilden. Zum Verschließen der unteren Öffnung des rohrförmigen Abschnitts 16 ist ein Verschlussstopfen 20 vorgesehen (Figuren 4 und 5). Die Ausbildung des Abschnitts 16 als durchgehendes Hohlrohr erleichtert eine Entformung nach dem Spritzgießen des Gehäuseunterteils 3a.

Um eine besonders einfache Verstellung des Gehäuseoberteils 3b in Bezug zum Gehäuseunterteil 3a zu bewirken, ist die Gehäuseführung 8 derart ausgebildet, dass eine Drehbewegung D um die Verbindungsachse A eine Verbreiterung des Spalts 17 bewirkt.

Hierzu ist bei dem dargestellten Ausführungsbeispiel der dem Gehäuseunterteil 3a zugeordnete Abschnitt 16 mit zwei Dornen 22 versehen, die bei der Montage in Eingriff mit als Führungskulissen für die Dorne 22 dienenden Schlitzen 21 in dem dem Gehäuseoberteil 3b zugeordneten rohrförmigen Abschnitt 15 gebracht werden. Durch eine entsprechende Anordnung der Schlitze 21, z. B. im Winkel von 45° zur Verbindungsachse A, wird erreicht, dass bei einer Drehbewegung D des Gehäuseoberteils 3b in Bezug zum Gehäuseunterteil 3a eine Drehbewegung D um die Verbindungsachse A in eine lineare Bewegung entlang der Verbindungsachse A bewirkt wird, mit der Folge, dass der Spalt 17 vergrößert wird. Alternativ können die Abschnitte 15, 16 auch mittels eines Gewindes gekoppelt sein.

Die Wirkstoffträger 2a, 2b weisen bei diesem ersten Ausführungsbeispiel eine - von einer Ausnehmung 25 bzw. Öffnung oder Ausschnitt unterbrochene - kreisförmige Umrandung 26 (Außenkontur) auf, deren Radius an der Anschlussstelle zu den Gehäuseteilen 3a, 3b gleich dem maximalen Radius des Gehäuseunterteils 3a und Gehäuseoberteils 3b ist. Die Ausnehmung 25 erstreckt sich jeweils radial bis über den Mittelpunkt des Wirkstoffträgers 2a, 2b in den Wirkstoffträger 2a, 2b hinein und ist U-förmig ausgebildet, wobei die Breite b bzw. der Radius des U-Grunds der Ausnehmung 25 an den Außenradius des zweiten Abschnitts 16 der teleskopartigen Verbindungsachse bzw. Gehäuseführung 8 angepasst ist.

Im Bereich der Ausnehmung 25 des Wirkstoffträgers 2a, 2b sind zwei parallel zu einem Radius des Wirkstoffträgers 2a, 2b und parallel zueinander verlaufende Begrenzungsflächen als Wirkstoffträgerführung 12 ausgebildet. Der Abstand der Wirkstoffträgerführung 12 ist dabei so gewählt, dass er mit geringem Spiel über den Außendurchmesser des rohrförmigen Abstands 16 des Gehäuseunterteils 3a passt. Ferner weist der Wirkstoffträger 2a, 2b einen bogenförmig ausgebildeten Anschlag 13 - den oben genannten U-Grund - auf, dessen Radius wie erwähnt dem Radius des rohrförmigen Abschnitts 16 des Gehäuseunterteils 3a entsprechend angepasst ist. Somit wird der Wirkstoffträger 2a, 2b durch die Wirkstoffträgerführung 12 geführt und durch den Anschlag 13 blockiert.

Zur Fixierung des unteren Wirkstoffträgers 2a im Gehäuse 3 kann in einem einfachsten Fall das Gehäuseunterteil 3a an seiner Oberseite 4 einen umlaufenden Haltering 23 aufweisen, der in eine passende Nut 24 an der Anschlussseite 7 des Wirkstoffträgers 2 eingreift und so den Wirkstoffträger 2 in seiner Position in Bezug zum Gehäuseunterteil 3a fixiert. Dies ist in den Figuren 3 und 4 gezeigt.

Der obere Wirkstoffträger 2b ist dagegen mit seiner Anschlussseite 7 an der Unterseite 5 des Gehäuseoberteils 3b so fixiert, dass er bei einer Bewegung des Gehäuseoberteils 3b nach oben mit angehoben wird.

Ein geeigneter oberer Wirkstoffträger 2b mit einer Ausnehmung 25 (für das Ausführungsbeispiel gemäß den Figuren 1 und 2) und ein hierzu passendes Gehäuseoberteil 3b ist in den Figuren 7 und 8 dargestellt. Dabei zeigt Figur 8 eine Draufsicht auf die Anschlussseite 7 des oberen Wirkstoffträgers 2b und Figur 7 eine Explosionsdarstellung der kompletten Wirkstoff-Abgabevorrichtung 1 von der Seite, wobei das Gehäuseoberteil 3b und das Gehäuseunterteil 3a jeweils im Schnitt dargestellt sind.

Das Gehäuseunterteil 3a und das Gehäuseoberteil 3b mit dem ersten Abschnitt 15 und den zweiten Abschnitt 16 der Gehäuseführung 8 sind in gleicher Weise aufgebaut, wie dies zuvor anhand der Figuren 4 bis 6 erläutert wurde. Lediglich der als Kulisse für die Dorne dienende Schlitz 21 weist hier in der oberen und unteren Stellung horizontal verlaufende Endabschnitte zur Fixierung des oberen Gehäuseteils 3b in den Endpositionen auf. Zudem ist in Figur 7 zusätzlich gezeigt, dass innerhalb des kalottenförmigen Innenraums des Gehäuseoberteils 3b in einem kurzen Abstand von der Kante der Unterseite 5 des Gehäuseoberteils 3b abschnittsweise entlang des Umfangs Wulstabschnitte 32 umlaufen, die nach innen hineinragen. Diese Wulstabschnitte 32 erstrecken sich in etwa über zwei gegenüberliegende Viertelkreissegmente. Die Wulstabschnitte 32 dienen als Verbindungselemente bzw. Rastelemente, um den oberen Wirkstoffträger 2b mit dem Gehäuseoberteil 3b zu verkoppeln.

Dementsprechend weist der obere Wirkstoffträger 2b auf seiner zum Gehäuseoberteil 3b weisenden Anschlussseite 7 einen Anschlussabschnitt oder "Anschlussflansch" auf. Der Durchmesser des Anschlussflansches ist etwas geringer als der Durchmesser der Au-βenkontur des im montierten Zustand von außen sichtbaren Teils (im Folgenden als "Sichtabschnitt" bezeichnet) des oberen Wirkstoffträgers 2b. An diesem Anschlussflansch sind ebenfalls teilweise entlang des Umfangs umlaufende Wulstabschnitte 30 angeordnet, wobei zwischen diesen Wulstabschnitten 30 und dem Sichtabschnitt des oberen Wirkstoffträgers 2b ein Abstand verbleibt, durch den eine partiell umlaufende Sicke 31 gebildet wird. Die Wulstabschnitte 32 an der Innenwand des Gehäuseoberteils 3b sind dabei so ausgebildet, dass sie genau in diese Sicke 31 an der Anschlussseite 7 des oberen Wirkstoffträgers 2b passen. Dabei erstrecken sich die Wulstabschnitte 30 an der Anschlussseite 7 des oberen Wirkstoffträgers 2b auch nur in etwa jeweils über zwei einander gegenüberliegende Viertelkreissegmente, was in der Draufsicht in Figur 8 zu sehen ist. Somit kann der obere Wirkstoffträger 2b mit der Anschlussseite 7 voraus von unten in die Kavität des Gehäuseoberteils 3b eingeschoben werden kann, wenn die Wulstabschnitte 30, 32 am oberen Wirkstoffträger 2b und im Gehäuseoberteil 3b passend gegeneinander verdreht sind. Wird dann der obere Wirkstoffträger 2b so gegenüber dem Gehäuseoberteil 3b um die Verbindungsachse A verdreht, das die Wulstabschnitte 30, 32 zumindest teilweise in Überdeckung kommen, wird der obere Wirkstoffträger 2b gegenüber einem Verrutschen des oberen Wirkstoffträgers 2b entlang der Verbindungsachse A vom Gehäuseoberteil 3b gesichert. Somit ist die gewünschte lösbare Kopplung zwischen dem oberen Wirkstoffträger 2b und dem Gehäuseoberteil 3b gegeben.

In dem dargestellten Ausführungsbeispiel ist der untere Wirkstoffträger 2a in gleicher Weise wie der obere Wirkstoffträger 2b ausgebildet, so dass die Wirkstoffträger 2a, 2b gegeneinander vertauscht werden können und nur ein Typ von Wirkstoffträger vorgehalten werden muss. Da auch beim unteren Wirkstoffträger 2a der Anschlussflansch an der Anschlussseite 7 in das kalottenförmige Gehäuseunterteil 3b eingreift, kann auf die Nut 24 an der Oberseite 4 des Gehäuseunterteils bei dieser Variante verzichtet werden.

Bei einer bevorzugten Variante befindet sich in einem Auslieferungszustand über der Kavität 10 der Wirkstoffträger 2a, 2b jeweils eine gasundurchlässige Schutzfolie (nicht dargestellt), die zum Beispiel mit einer Oberseite der Umrandung 26 des Wirkstoffträgers 2a, 2b verklebt ist. Der Verbraucher kann dann zunächst die Wirkstoffträger 2a, 2b aus dem Gehäuse 3 entnehmen, die Schutzfolie abziehen und die Wirkstoffträger 2a, 2b wieder im Gehäuse 3 einsetzen. Zur Entnahme der Wirkstoffträger 2a, 2b werden diese jeweils zunächst in Richtung der Verbindungsachse A verschoben, wobei zuvor der obere Wirkstoffträger 2b zuerst wie oben erläutert relativ zum Gehäuseoberteil verdreht wird, um ihn freizugeben. Anschließend kann der Wirkstoffträger 2a, 2b durch eine Bewegung in eine Richtung senkrecht zur Verbindungsachse A aus dem Spalt 17 gezogen werden. Das Einsetzen der Wirkstoffträgers 2a, 2b erfolgt auf umgekehrten Weg. In gleicher Weise kann bei dem in den Figuren 1 bis 8 gezeigten Ausführungsbeispielen ein Wechsel des Wirkstoffträgers 2a, 2b vorgenommen werden, wenn der Vorrat an Duftstoffen im oberen bzw. unteren Wirkstoffträger 2a, 2b erschöpft ist.

Die Maße des Gehäuses 3 und der Wirkstoffträger 2a, 2b sind bei den dargestellten bevorzugten Ausführungsbeispielen wie folgt: Der Durchmesser des Außen-Querschnitts des Gehäuses 3 und der Wirkstoffträger 2a, 2b senkrecht zur Verbindungsachse A beträgt je nach Ausführungsbeispiel zwischen 7,5 cm und 8 cm. Die Höhe des Gehäuses 3 beträgt, bei einer Kugelform wegen der Abflachung zur Bildung der Standfläche 18, dann ca. 7,2 cm. Die Dicke (bzw. Höhe) der scheibenförmigen Wirkstoffträger 2a, 2b ist so gewählt, dass eine Füllhöhe in der Kavität von ca. 4 mm gewährleistet ist. Die Breite des Spalts ist zwischen einem Minimalwert von ca. 5 mm und einem Maximalwert von ca. 10 mm bis ca. 15 mm einstellbar.

Die Figuren 9 und 10 zeigen ein weiteres Ausführungsbeispiel einer erfindungsgemäßen Wirkstoff-Abgabevorrichtung 1. In diesem Ausführungsbeispiel weisen die Wirkstoffträger 2a', 2b' keine Aussparung auf, sondern nur ein mittleres Loch, dessen Innendurchmesser so gewählt ist, dass die Wirkstoffträger 2a, 2b über den zweiten Abschnitt 16 der Gehäuseführung 8 im Gehäuseunterteil 3a' geschoben werden können. Das heißt, sowohl der untere Wirkstoffträger 2a' als auch der obere Wirkstoffträger 2b' weisen eine kreisrunde Außenkontur auf. Der Durchmesser dieser Außenkontur am "Sichtabschnitt" ist wiederum so gewählt, dass er dem Außendurchmesser an der jeweils zum Spalt 17 weisenden Oberseite 4 des Gehäuseunterteils 3a' bzw. der Unterseite 5 des Gehäuseoberteils 3b' entspricht.

Um den oberen Wirkstoffträger 2b' mit dem Gehäuseoberteil 3b' an dessen Unterseite 5 zu verkoppeln, weist das Gehäuseoberteil 3b' auch hier im Inneren des Gehäuses in einem kurzen Abstand oberhalb der unteren Kante eine Wulst 35 auf, wobei jedoch, anders als bei dem Ausführungsbeispiel gemäß Figur 7, diese Wulst 35 komplett kreisförmig umläuft. Entsprechend ist der Anschlussflansch an der Anschlussseite 7 des oberen Wirkstoffträgers 2b' mit einer umlaufenden Wulst 33 ausgebildet, so dass zwischen der umlaufenden Wulst 33 und dem Sichtabschnitt eine kreisförmig umlaufende Sicke 34 ausgebildet ist. Im Bereich der Wulst 33 ist der obere Wirkstoffträger 2b' konisch zulaufend ausgebildet, d. h. der Umfang verringert sich in Richtung auf die Unterseite 5 des Gehäuseoberteils 3b'. Dadurch kann der obere Wirkstoffträger 2b' mit etwas Druck in die Unterseite 5 des Gehäuseoberteils 3b' eingepresst werden, wobei die Wulst 35 über die Wulst 33 in die Sicke 34 einschnappt. Der obere Wirkstoffträger 2b' ist dann um die Verbindungsachse A drehbar, aber im Normalfall für den Verbraucher nicht lösbar mit dem Gehäuseoberteil 3b' verbunden.

Der untere Wirkstoffträger 2a' ist wieder identisch zu dem oberen Wirkstoffträger 2b' aufgebaut, so dass auch bei diesem Ausführungsbeispiel nur ein Typ von Wirkstoffträger gebraucht wird. Um den unteren Wirkstoffträger 2a' ebenfalls fest im Gehäuseunterteil 3a' zu halten, ist hier auch im Gehäuseunterteil 3a' in einem kurzen Abstand von der Kante an der Oberseite 4 des Gehäuseunterteils 3a' innen eine umlaufende Wulst 35 angeordnet.

Bei diesem Ausführungsbeispiel erfolgt die Montage der Wirkstoff-Abgabevorrichtung 1 in der Weise, dass zunächst der untere Wirkstoffträger 2a' über den zweiten Abschnitt 16 der Gehäuseführung 8 am Gehäuseunterteil 3a' aufgeschoben wird, und zwar so, dass die Anschlussseite 7 des unteren Wirkstoffträgers 2a' zum Gehäuseunterteil 3a' weist. Anschließend wird dann der obere Wirkstoffträger 2b' kopfüber, d. h. mit seiner Anschlussseite 7 nach oben weisend, ebenfalls über den zweiten Abschnitt 16 der Gehäuseführung 8 geschoben. Danach wird zur Endmontage des Gehäuses 3' das Gehäuseoberteil 3b' aufgesetzt, wobei der erste Abschnitt 15 am Gehäuseoberteil 3b' in den zweiten Abschnitt 16 der Gehäuseführung 8 eingeschoben wird, so dass die Dorne 22 im Inneren des zweiten Abschnitts 16 in die Schlitze 21 des ersten Abschnitts 15 eingreifen. Dabei wird das Gehäuseoberteil 3b' so fest auf das Gehäuseunterteil 3a' gepresst, dass hierbei zwangsläufig auch die Wulst 35 im unteren Gehäuseteil 3a' in die Sicke 34 im unteren Wirkstoffträger 2a' und die Wulst 35 im oberen Gehäuseoberteil 3b' in die Sicke 34 des oberen Wirkstoffträgers 2b' einschnappt. Ein Verschließen der Kavitäten 10 mit einer gasundurchlässigen Membran ist hierbei nicht nötig, da im komplett zusammengeschobenen Zustand (d. h. wie bei der in Figur 1 dargestellten Position I) und der kreisförmigen Außenkontur der Wirkstoffträger 2a', 2b' keinerlei Abdampföffnung mehr vorhanden ist. Diese wird erst gebildet, wenn das obere Gehäuseteil 3b' gegenüber dem unteren Gehäuseteil 3a' verlagert und der Spalt a vergrößert wird, so dass eine Öffnung zwischen den beiden Wirkstoffträgern 2a', 2b' entsteht.

Sowohl bei dem Ausführungsbeispiel gemäß Figur 9 als auch bei dem Ausführungsbeispiel gemäß Figur 7 sind die Wirkstoffträger 2a, 2b, 2a', 2b' durch die in das Gehäuseunterteil 3a, 3b, 3a', 3b' hineinragenden Anschlussflansche jeweils dicker ausgebildet als Wirkstoffträger ohne einen solchen Flansch wie z. B. gemäß Figur 3. Dieser zusätzliche Raum innerhalb der Wirkstoffträger 2a, 2b, 2a', 2b' wird genutzt, um die Kavität 10 jeweils tiefer auszubilden, wie dies in den Figuren 7 und 9 durch die gestrichelten Linien dargestellt ist. Bei diesem Ausführungsbeispiel ist somit das Wirkstoffvolumen größer als bei flachen Scheiben, so dass eine längere Haltbarkeit gegeben ist.

Um auch im Ausführungsbeispiel gemäß den Figuren 9 und 10 ein Austauschen der Wirkstoffträger 2a', 2b' zu erlauben, könnten diese im Prinzip mit Verbindungselementen analog zu dem Ausführungsbeispiel gemäß Figur 7 und 8 ausgebildet sein. Das heißt, dass beispielsweise anstelle der umlaufenden Wulste nur Wulstabschnitte vorhanden sind, die sich jeweils über ein Viertelkreissegment erstrecken. In diesem Fall kann dann wegen der kreisförmigen Außenkontur der Austausch der Wirkstoffträger so erfolgen, dass das Gehäuseoberteil 3b' vollständig vom Gehäuseunterteil 3a' entfernt wird.

Wie oben bereits erwähnt, kann anstelle eines gelförmigen Trägerstoffs oder eines Trägers in Form eines Vlieses oder dergleichen bzw. eines flüssigen Wirkstoffs mit einer Membran an der Oberseite des Wirkstoffträgers auch ein Wirkstoffbehälter in die Kavität des Wirkstoffträgers eingesetzt werden.

In Figur 11 ist hierzu eine schematische Draufsicht und darüber eine Schnittdarstellung entlang der in der Draufsicht eingezeichneten Schnittlinie S₁ eines Wirkstoffbehälters 40 dargestellt. Der Wirkstoffbehälter 40 ist mit seinen äußeren Abmessungen hier an die Innenabmessungen der Kavität 10 der Wirkstoffträger 2a, 2b gemäß den Figuren 7 und 8 angepasst, d. h. er ist in der Draufsicht im Wesentlichen U-förmig ausgebildet. Der Wirkstoffbehälter 40 weist ein oben offenes topfförmiges Gehäuse 41 zur Aufnahme des Wirkstoffs, ggf. mit einem beispielsweise flüssigen oder gelartigen Trägerstoff, auf, an dem oben seitlich ein rundum laufender Flansch 44 angeformt ist. Dieses Gehäuse 41 kann vorteilhaft kostengünstig aus Tiefziehfolie hergestellt sein, welche gerade noch so dick ist, dass das Gehäuse 41 unter normalen Temperaturbedingungen seine Form behält. Nach der Befüllung des Gehäuses 41 wird dieses mit einer vorzugsweise semipermeablen Membran 42 abgedeckt, welche am Rand mit dem Flansch 44 dicht verbunden, beispielsweise verklebt oder verschweißt wird. Zusätzlich kann diese Membran 42 zunächst noch mit einer Schutzfolie (nicht dargestellt) abgedeckt sein, welche vom Verbraucher abgezogen werden kann, wenn der Wirkstoffbehälter 20 in den Wirkstoffträger 2a, 2b eingesetzt und in Betrieb genommen werden soll.

An der Außenwand des Wirkstoffbehälters 40 verläuft entlang zumindest eines Teils des Umfangs eine Wulst 43. Diese sorgt für eine Klemmung an der Innenseite der radial äußeren Wand der Kavität 10. Gegebenenfalls kann an der Innenseite der radial äußeren Wand der Kavität 10 auch eine entsprechende, zumindest teilweise umlaufende Wulst angeordnet sein (nicht dargestellt), die mit der Wulst 43 am Wirkstoffbehälter 40 zusammenwirkt, so dass dieser in die Kavität 10 eingeclipst werden kann.

Es wird abschließend noch einmal darauf hingewiesen, dass es sich bei den in den Figuren dargestellten Wirkstoff-Abgabevorrichtungen, Gehäusen und Wirkstoffträgern nur um Ausführungsbeispiele handelt, welche vom Fachmann in vielfacher Hinsicht variiert werden können, ohne den Rahmen der Erfindung zu verlassen. So sind insbesondere auch verschiedenste Kombinationen der gezeigten Bestandteile der Wirkstoff-Abgabevorrichtung möglich. Ebenso sind auch Kombinationen mit den in der DE 10 2010 051 409 und der EP 11 008 739.2 gezeigten Ausführungsbeispielen möglich. Insbesondere können auch mehr als zwei Wirkstoffträger eingesetzt werden, beispielsweise ein oberer Wirkstoffträger, der mit dem Gehäuseoberteil gekoppelt ist, und weitere auf dem unteren Wirkstoffträger aufliegende andere Wirkstoffträger, analog zu dem Vorschlag in der DE 10 2010 051 409. Es wird außerdem der Vollständigkeit halber darauf hingewiesen, dass die Verwendung des unbestimmten Artikels "ein" bzw. "eine" nicht ausschließt, dass die betreffenden Merkmale auch mehrfach vorhanden sein können.

### Bezugszeichenliste

- 1: Wirkstoff-Abgabevorrichtung
- 2a, 2a': unterer Wirkstoffträger
- 2b, 2b': oberer Wirkstoffträger
- 3, 3': Gehäuse
- 3a, 3a': Gehäuseunterteil
- 3b, 3b': Gehäuseoberteil
- 4: Oberseite des Gehäuseunterteils
- 5: Unterseite des Gehäuseoberteils
- 6: Wirkstoffabgabeseite des Wirkstoffträgers
- 7: Anschlussseite des Wirkstoffträgers
- 8: Gehäuseführung
- 9: Außengewinde
- 10: Kavität
- 12: Wirkstoffträgerführung
- 13: Anschlag
- 14: Gehäuseinneres
- 15: erster Abschnitt
- 16: zweiter Abschnitt
- 17: Spalt
- 18: Standfläche
- 19, 19': Ausdampföffnung
- 20: Verschlussstopfen
- 21: Schlitz
- 22: Dorn
- 23: Haltering
- 24: Nut
- 25: Ausnehmung
- 26: Umrandung
- 27: Trägerstoff
- 30: Verbindungselement / Wulstabschnitt
- 31: Verbindungselement / Sicke
- 32: Verbindungselement / Wulstabschnitt
- 33: Verbindungselement / Wulst
- 34: Verbindungselement / Sicke
- 35: Verbindungselement / Wulst
- 40: Wirkstoffbehälter
- 41: Gehäuse
- 42: Membran
- 43: Halteelement / Wulst
- 44: Flansch
- a: Abstand
- a': vergrößerter Abstand
- b: Breite
- A: Verbindungsachse
- B: Montageachse
- D: Drehbewegung
- S₁: Schnittlinie
- I: erste Position
- II: zweite Position

## Patentansprüche

1. Wirkstoff-Abgabevorrichtung (1) zur Abgabe von gasförmigen Wirkstoffen, insbesondere von Duftstoffen, in die Umgebung, mit
- einem Gehäuse (3, 3') mit einem Gehäuseunterteil (3a, 3a') und einem Gehäuseoberteil (3b, 3b'), wobei das Gehäuseoberteil (3b, 3b') und das Gehäuseunterteil (3a, 3a') unter Bildung eines Spalts (17) mit einem Abstand (a, a') voneinander angeordnet sind,
- einem dem Gehäuseunterteil (3a, 3a') zugeordneten unteren Wirkstoffträger (2a, 2a'),
- einem dem Gehäuseoberteil (3b, 3b') zugeordneten oberen Wirkstoffträger (2b, 2b'), wobei der untere Wirkstoffträger (2a, 2a') und der obere Wirkstoffträger (2b, 2b') jeweils zumindest teilweise in dem Spalt (17) angeordnet sind und der obere Wirkstoffträger (2b, 2b') mit dem Gehäuseoberteil (3a) gekoppelt ist.

2. Wirkstoff-Abgabevorrichtung (1) nach Anspruch 1, **dadurch gekennzeichnet, dass** der untere Wirkstoffträger (2a, 2a') an eine zum Gehäuseoberteil (3b, 3b') weisende Oberseite (4) des Gehäuseunterteils (3a, 3a') angrenzt oder in diese eingreift und der obere Wirkstoffträger (2b, 2b') an eine zum Gehäuseunterteil (3a, 3a') weisende Unterseite (5) des Gehäuseoberteils (3b, 3b') angrenzt oder in diese eingreift.

3. Wirkstoff-Abgabevorrichtung (1) nach Anspruch 1 oder 2, **dadurch gekennzeichnet, dass** der untere Wirkstoffträger (2a, 2a') und der obere Wirkstoffträger (2b, 2b') jeweils, vorzugsweise zumindest in ihren zueinander weisenden Seiten, eine Kavität (10) zur Aufnahme des Wirkstoffs und gegebenenfalls eines den Wirkstoff enthaltenden Trägerstoffs (27) und/oder zur Aufnahme eines Wirkstoffbehälters (30) aufweisen, welcher den Wirkstoff und gegebenenfalls den Wirkstoff enthaltenden Trägerstoff enthält.

4. Wirkstoff-Abgabevorrichtung (1) nach einem der Ansprüche 1 bis 3, **dadurch gekennzeichnet, dass** der obere Wirkstoffträger (2b, 2b') an der Unterseite (5) des Gehäuseoberteils (3b, 3b') mit diesem, vorzugsweise lösbar, verbunden ist.

5. Wirkstoff-Abgabevorrichtung (1) nach Anspruch 4, **dadurch gekennzeichnet, dass** der obere Wirkstoffträger (2b, 2b') formschlüssig mit dem Gehäuseoberteil (3b, 3b') verbunden ist.

6. Wirkstoff-Abgabevorrichtung (1) nach Anspruch 5, **dadurch gekennzeichnet, dass** der obere Wirkstoffträger (2b) an einer zur Unterseite (5) des Gehäuseoberteils (3b) weisenden Anschlussseite (7) mechanische Verbindungselemente (30, 32, 33) aufweist, welche mit mechanischen Verbindungselementen (31, 34) an oder im Gehäuseoberteil (3b) so zusammenwirken, dass der obere Wirkstoffträger (2b) in einer ersten Drehstellung relativ zum Gehäuseoberteil (3b) bezüglich einer Drehung um eine zwischen dem Gehäuseoberteil (3b) und dem Gehäuseunterteil (3a) verlaufende Verbindungsachse (A) am Gehäuseoberteil (3b) gehalten wird und in einer zweiten Drehstellung von diesem lösbar ist.

7. Wirkstoff-Abgabevorrichtung (1) nach einem der Ansprüche 1 bis 6, **dadurch gekennzeichnet, dass** der Abstand (a, a') vom Gehäuseoberteil (3b, 3b') zu dem Gehäuseunterteil (3a, 3a') durch Verlagern des Gehäuseoberteils (3b) relativ zum Gehäuseunterteil (3a) entlang einer Verbindungsachse (A) variierbar ist.

8. Wirkstoff-Abgabevorrichtung (1) nach Anspruch 7, **gekennzeichnet durch** eine, vorzugsweise im Inneren (14) des Gehäuses (3) angeordnete, Gehäuseführung (8), **durch** die das Gehäuseunterteil (3a, 3a') und das Gehäuseoberteil (3b, 3b') so gekoppelt sind, dass zum Verlagern eine lineare Bewegung des Gehäuseoberteils (3b, 3b') relativ zum Gehäuseunterteil (3a, 3a') entlang der Verbindungsachse (A) durchführbar ist.

9. Wirkstoff-Abgabevorrichtung (1) nach einem der Ansprüche 1 bis 7, **dadurch gekennzeichnet, dass** zumindest einer der Wirkstoffträger (2a, 2b) durch ein Verlagern des Wirkstoffträgers (2a, 2b) relativ zum Gehäuse (3) quer zur Verbindungsachse (A) in oder aus dem Spalt (17) bringbar ist.

10. Wirkstoff-Abgabevorrichtung (1) nach Anspruch 9, **dadurch gekennzeichnet, dass** der untere und/oder obere Wirkstoffträger (2a, 2b) eine Wirkstoffträgerführung (12) aufweisen, um den betreffenden Wirkstoffträger (2a, 2b) beim Einschieben in den Spalt (17) zu führen, und der betreffende Wirkstoffträger (2a, 2b) wenigstens einen Anschlag (13) aufweist, um eine Bewegung in den Spalt (17) zu begrenzen, wobei die Wirkstoffträgerführung (12) und/oder der Anschlag (13) mit der Gehäuseführung (8) des Gehäuses (3) zusammenwirken.

11. Wirkstoff-Abgabevorrichtung (1) nach einem der Ansprüche 8 bis 10, **dadurch gekennzeichnet, dass** die Gehäuseführung (8) einen ersten Abschnitt (15) und einen zweiten Abschnitt (16) umfasst, wobei der erste und/oder zweite Abschnitt (15, 16) sich in Richtung der Verbindungsachse (A) aus dem Gehäuseunterteil (3a, 3a') und/oder Gehäuseoberteil (3b, 3b') erstrecken.

12. Gehäuse (3, 3') für eine Wirkstoff-Abgabevorrichtung (1) nach einem der Ansprüche 1 bis 11.

13. Wirkstoffträger (2a, 2b, 2a', 2b') für eine Wirkstoff-Abgabevorrichtung (1) nach einem der Ansprüche 1 bis 11.

14. Wirkstoffbehälter (40) enthaltend einen Wirkstoff, wobei der Wirkstoffbehälter (40) zur Aufnahme in einem Wirkstoffträger (2a, 2b, 2a', 2b') nach Anspruch 13 ausgebildet ist.
